# EUROPEAN PATENT APPLICATION

(11) **EP 0 669 397 A1**
(43) Date of publication of application: **30.08.1995**
(21) Application number: 94908100.4
(22) Date of filing: 12.08.1993
(51) Int. Cl.: C12P 21/02

(54) **NOVEL PROTEIN AND GENE CODING FOR THE SAME**

(30) Priority: 12.08.1992 JP 215017/92
(71) Applicant: MITSUBISHI KASEI CORPORATION, Tokyo 100 (JP)
(72) Inventor: MISHINA, Masayoshi, Niigata-shi, Niigata 950-21 (JP)
(74) Representative: ter Meer, Nicolaus, Dipl.-Chem., Dr.
(86) International application number: JP9301143
(87) International publication number: WO9404698

(57) **Abstract**

A gene coding for a modification of an NMDA type glutamate receptor obtained from a mouse cerebellum is prepared by the site-specific mutagenesis of the glutamate receptor cDNA. The above modification is prepared by synthesizing an mRNA from the above gene and introducing the mRNA into the ovocyte of Xenopus laevis to effect translation. The modification is useful for clarifying the mechanism of neurotransmission across synapses, that of development of synaptic plasticity serving as the base of memory and learning and that of neurocyte necrosis occurring in the morbidity caused by cerebral ischemia and status epilepticus, for understanding the mechanism of neurotransmission in the central nervous system and the superstructure and morbidity of the brain, and for creating the therapy of genetic diseases and novel medicines.

### SEQENCE LISTING

(1) GENERAL INFORMATION:
   (i) APPLICANT: Mitsubishi Chemical Corporation
   (ii) TITLE OF INVENTION: Novel Proteins and Genes Coding the Same
   (iii) NUMBER OF SEQUENCES:3
   (iv) CORRESPONDENCE ADDRESS:
      (A) ADDRESSEE:
      (B) STREET:
      (C) CITY:
      (D) STATE:
      (E) COUNTRY:
      (F) ZIP:
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME:
      (B) REGISTRATION NUMBER:
      (C) REFERENCE/DOCKET NUMBER:
   (ix) TELECOMMUNICATION INFORMATION:
      (A) TELEPHONE:
      (B) TELEFAX:
(2) INFORMATION FOR SEQ ID NO:l:
   (i) SEQUENCE CHARACTERISTICS:
      (A) LENGTH:4368
      (B) TYPE: nucleic acid
      (C) STRANDEDNESS: double
      (D) TOPOLOGY: linear
   (ii) MOLECULAR TYPE: cDNA
   (vi) ORIGINAL SOURCE: mouse
      (A) ORGANISM: brain
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 2760
      (B) TYPE: nucleic acid
      (C) STRANDEDNESS: double
      (D) TOPOLOGY: linear
   (ii) MOLECULAR TYPE: cDNA
   (vi) ORIGINAL SOURCE: mouse
      (A) ORGANISM: brain
   (xi) SEQUENCE DESCRIPTION: SEQ ID NO:2:
   CGC GCT GCC TGC GAC CCC AAG ATT GTC AAC ATC GGC GCG GTG CTG AGC      48
   Arg Ala Ala Cys Asp Pro Lys Ile Val Asn Ile Gly Ala Val Leu Ser
    1               5                  10                  15
   ACG CGC AAG CAC GAG CAG ATG TTC CGC GAG GCA GTA AAC CAG GCC AAT      96
   Thr Arg Lys His Glu Gln Met Phe Arg Glu Ala Val Asn Gln Ala Asn
               20                  25                  30
   AAG CGA CAC GGC TCT TGG AAG ATA CAG CTC AAC GCC ACT TCT GTC ACC     144
   Lys Arg His Gly Ser Trp Lys Ile Gln Leu Asn Ala Thr Ser Val Thr
           35                  40                  45
   CAC AAG CCC AAC GCC ATA CAG ATG GCC CTG TCA GTG TGT GAG GAC CTC     192
   His Lys Pro Asn Ala Ile Gln Met Ala Leu Ser Val Cys Glu Asp Leu
       50                  55                  60
   ATC TCT AGC CAG GTC TAC GCT ATC CTA GTT AGT CAC CCG CCT ACT CCC     240
   Ile Ser Ser Gln Val Tyr Ala Ile Leu Val Ser His Pro Pro Thr Pro
   65                  70                  75                  80
   AAC GAC CAC TTC ACT CCC ACC CCT GTC TCC TAC ACA GCT GGC TTC TAC     288
   Asn Asp His Phe Thr Pro Thr Pro Val Ser Tyr Thr Ala Gly Phe Tyr
                   85                  90                  95
   AGA ATC CCC GTC CTG GGG CTG ACT ACC CGA ATG TCC ATC TAC TCT GAC     336
   Arg Ile Pro Val Leu Gly Leu Thr Thr Arg Met Ser Ile Tyr Ser Asp
               100                 105                 110
   AAG AGC ATC CAC CTG AGC TTC CTT CGC ACC GTA CCA CCC TAC TCC CAC     384
   Lys Ser Ile His Leu Ser Phe Leu Arg Thr Val Pro Pro Tyr Ser His
           115                 120                 125
   CAG TCC AGC GTC TGG TTT GAG ATG ATG CGC GTC TAC AAC TGG AAC CAT     432
   Gln Ser Ser Val Trp Phe Glu Met Met Arg Val Tyr Asn Trp Asn His
       130                 135                 140
   ATC ATC CTG CTG GTC AGC GAT GAC CAC GAG GGC CGG GCA GCG CAG AAG     480
   Ile Ile Leu Leu Val Ser Asp Asp His Glu Gly Arg Ala Ala Gln Lys
   145                 150                 155                 160
   CGC CTG GAG ACG TTG CTG GAG GAG CGT GAG TCC AAG GCA GAG AAG GTG     528
   Arg Leu Glu Thr Leu Leu Glu Glu Arg Glu Ser Lys Ala Glu Lys Val
                   165                 170                 175
   CTG CAG TTT GAC CCA GGA ACC AAG AAT GTG ACG GCT CTG CTG ATG GAA     576
   Leu Gln Phe Asp Pro Gly Thr Lys Asn Val Thr Ala Leu Leu Met Glu
               180                 185                 190
   GCC CGG GAC CTG GAA GCC CGG GTC ATC ATC CTT TCT GCA AGC GAG GAC      624
   Ala Arg Asp Leu Glu Ala Arg Val Ile Ile Leu Ser Ala Ser Glu Asp
           195                     200                     205
   GAC GCT GCC ACC GTA TAC CGC GCA GCC GCG ATG CTG AAC ATG ACT GGC      672
   Asp Ala Ala Thr Val Tyr Arg Ala Ala Ala Met Leu Asn Met Thr Gly
       210                     215                     220
   TCT GGG TAC GTG TGG CTC GTC GGG GAG CGC GAG ATC TCT GGG AAT GCC      720
   Ser Gly Tyr Val Trp Leu Val Gly Glu Arg Glu Ile Ser Gly Asn Ala
   225                     230                     235                 240
   CTG CGC TAC GCT CCT GAC GGC ATC ATC GGA CTT CAG CTA ATC AAC GGC      768
   Leu Arg Tyr Ala Pro Asp Gly Ile Ile Gly Leu Gln Leu Ile Asn Gly
                   245                     250                     255
   AAG AAC GAG TCG GCC CAC ATC AGT GAC GCT GTG GGC GTG GTG GCA CAG      816
   Lys Asn Glu Ser Ala His Ile Ser Asp Ala Val Gly Val Val Ala Gln
               260                     265                     270
   GCA GTC CAC GAG CTC CTA GAA AAG GAG AAC ATC ACT GAT CCA CCG CGG      864
   Ala Val His Glu Leu Leu Glu Lys Glu Asn Ile Thr Asp Pro Pro Arg
           275                     280                     285
   GGT TGC GTG GGC AAC ACC AAC ATC TGG AAG ACA GGA CCA CTG TTC AAG      912
   Gly Cys Val Gly Asn Thr Asn Ile Trp Lys Thr Gly Pro Leu Phe Lys
       290                     295                     300
   AGG GTG CTG ATG TCT TCC AAG TAT GCA GAT GGA GTG ACT GGC CGT GTG      960
   Arg Val Leu Met Ser Ser Lys Tyr Ala Asp Gly Val Thr Gly Arg Val
   305                     310                     315                 320
   GAA TTC AAT GAG GAT GGG GAC CGG AAG TTT GCC AAC TAT AGT ATC ATG     1008
   Glu Phe Asn Glu Asp Gly Asp Arg Lys Phe Ala Asn Tyr Ser Ile Met
                   325                     330                     335
   AAC CTG CAG AAC CGC AAG CTG GTG CAA GTG GGC ATC TAC AAT GGT ACC     1056
   Asn Leu Gln Asn Arg Lys Leu Val Gln Val Gly Ile Tyr Asn Gly Thr
               340                     345                     350
   CAT GTC ATC CCA AAT GAC AGG AAG ATC ATC TGG CCA GGA GGA GAG ACA     1104
   His Val Ile Pro Asn Asp Arg Lys Ile Ile Trp Pro Gly Gly Glu Thr
           355                     360                     365
   GAG AAG CCT CGA GGA TAC CAG ATG TCC ACC AGA CTA AAG ATA GTG ACA     1152
   Glu Lys Pro Arg Gly Tyr Gln Met Ser Thr Arg Leu Lys Ile Val Thr
       370                     375                     380
   ATC CAC CAA GAA CCC TTC GTG TAT GTC AAG CCC ACA ATG AGT GAT GGC     1200
   Ile His Gln Glu Pro Phe Val Tyr Val Lys Pro Thr Met Ser Asp Gly
   385                     390                     395                 400
   ACA TGC AAA GAG GAG TTC ACA GTC AAT GGT GAC CCT GTC AAG AAG GTG     1248
   Thr Cys Lys Glu Glu Phe Thr Val Asn Gly Asp Pro Val Lys Lys Val
                   405                     410                     415
   ATC TGT ACG GGG CCT AAT GAC ACA TCC CCA GGA AGC CCA CGT CAC ACA     1296
   Ile Cys Thr Gly Pro Asn Asp Thr Ser Pro Gly Ser Pro Arg His Thr
               420                     425                     430
   GTG CCC CAG TGC TGT TAT GGC TTC TGC GTT GAC CTG CTC ATC AAG CTG     1344
   Val Pro Gln Cys Cys Tyr Gly Phe Cys Val Asp Leu Leu Ile Lys Leu
       435                 440                 445
   GCA CGG ACC ATG AAT TTT ACC TAC GAG GTG CAC CTT GTG GCA GAT GGC     1392
   Ala Arg Thr Met Asn Phe Thr Tyr Glu Val His Leu Val Ala Asp Gly
       450                 455                 460
   AAG TTT GGC ACA CAG GAG CGG GTA AAC AAC AGC AAC AAA AAG GAG TGG     1440
   Lys Phe Gly Thr Gln Glu Arg Val Asn Asn Ser Asn Lys Lys Glu Trp
   465                 470                 475                 480
   AAC GGA ATG ATG GGA GAG CTG CTC AGT GGT CAA GCA GAC ATG ATC GTG     1488
   Asn Gly Met Met Gly Glu Leu Leu Ser Gly Gln Ala Asp Met Ile Val
                   485                 490                 495
   GCT CCA CTG ACC ATT AAC AAT GAG CGT GCG CAG TAC ATA GAG TTC TCC     1536
   Ala Pro Leu Thr Ile Asn Asn Glu Arg Ala Gln Tyr Ile Glu Phe Ser
               500                 505                 510
   AAG CCC TTC AAG TAC CAG GGC CTG ACC ATT CTG GTC AAG AAG GAG ATC     1584
   Lys Pro Phe Lys Tyr Gln Gly Leu Thr Ile Leu Val Lys Lys Glu Ile
           515                 520                 525
   CCT CGG AGC ACA CTG GAC TCA TTC ATG CAG CCC TTT CAG AGC ACA CTG     1632
   Pro Arg Ser Thr Leu Asp Ser Phe Met Gln Pro Phe Gln Ser Thr Leu
       530                 535                 540
   TGG CTG CTG GTG GGG CTG TCA GTT CAT GTG GTG GCC GTG ATG CTG TAC     1680
   Trp Leu Leu Val Gly Leu Ser Val His Val Val Ala Val Met Leu Tyr
   545                 550                 555                 560
   CTG CTG GAC CGC TTC AGT CCC TTT GGC CGA TTT AAG GTG AAC AGC GAG     1728
   Leu Leu Asp Arg Phe Ser Pro Phe Gly Arg Phe Lys Val Asn Ser Glu
                   565                 570                 575
   GAG GAG GAG GAG GAT GCA CTG ACC CTG TCC TCT GCC ATG TGG TTT TCC     1776
   Glu Glu Glu Glu Asp Ala Leu Thr Leu Ser Ser Ala Met Trp Phe Ser
               580                 585                 590
   TGG GGC GTC CTG CTC CAG TCT GGC ATT GGG GAA GGT GCC CCC CGG AGT     1824
   Trp Gly Val Leu Leu Gln Ser Gly Ile Gly Glu Gly Ala Pro Arg Ser
           595                 600                 605
   TTC TCT GCT CGT ATC CTA GGC ATG GTG TGG GCT GGT TTT GCC ATG ATC     1872
   Phe Ser Ala Arg Ile Leu Gly Met Val Trp Ala Gly Phe Ala Met Ile
       610                 615                 620
   ATC GTG GCT TCC TAC ACT GCC AAC CTG GCA GCC TTC CTG GTG CTG GAT     1920
   Ile Val Ala Ser Tyr Thr Ala Asn Leu Ala Ala Phe Leu Val Leu Asp
   625                 630                 635                 640
   AGG CCT GAG GAG CGC ATC ACA GGC ATC AAT GAC CCC AGG CTC AGA AAC     1968
   Arg Pro Glu Glu Arg Ile Thr Gly Ile Asn Asp Pro Arg Leu Arg Asn
                   645                 650                 655
   CCC TCA GAC AAG TTC ATC TAT GCA ACT GTA AAA CAG AGC TCT GTG GAT     2016
   Pro Ser Asp Lys Phe Ile Tyr Ala Thr Val Lys Gln Ser Ser Val Asp
               660                 665                 670
   ATC TAC TTC CGG AGG CAG GTG GAG TTG AGC ACC ATG TAC CGG CAC ATG     2064
   Ile Tyr Phe Arg Arg Gln Val Glu Leu Ser Thr Met Tyr Arg His Met
           675                 680                 685
   GAG AAG CAC AAT TAT GAG AGT GCA GCT GAG GCC ATC CAG GCT GTG CGG     2112
   Glu Lys His Asn Tyr Glu Ser Ala Ala Glu Ala Ile Gln Ala Val Arg
       690                 695                 700
   GAC AAC AAG CTC CAT GCC TTC ATC TGG GAC TCA GCT GTG CTG GAG TTT     2160
   Asp Asn Lys Leu His Ala Phe Ile Trp Asp Ser Ala Val Leu Glu Phe
   705                 710                 715                 720
   GAG GCT TCA CAG AAG TGC GAT CTG GTG ACC ACG GGT GAG CTG TTC TTC     2208
   Glu Ala Ser Gln Lys Cys Asp Leu Val Thr Thr Gly Glu Leu Phe Phe
                   725                 730                 735
   CGC TCC GGC TTT GGC ATC GGC ATG CGC AAG GAC AGC CCC TGG AAG CAA     2256
   Arg Ser Gly Phe Gly Ile Gly Met Arg Lys Asp Ser Pro Trp Lys Gln
               740                 745                 750
   AAT GTG TCC CTG TCC ATA CTC AAG TCC CAT GAG AAT GGC TTC ATG GAA     2304
   Asn Val Ser Leu Ser Ile Leu Lys Ser His Glu Asn Gly Phe Met Glu
           755                 760                 765
   GAC CTG GAT AAG ACA TGG GTT CGG TAT CAA GAA TGT GAC TCC CGC AGC     2352
   Asp Leu Asp Lys Thr Trp Val Arg Tyr Gln Glu Cys Asp Ser Arg Ser
       770                 775                 780
   AAT GCC CCT GCC ACC CTC ACT TTT GAG AAC ATG GCA GGG GTC TTC ATG     2400
   Asn Ala Pro Ala Thr Leu Thr Phe Glu Asn Met Ala Gly Val Phe Met
   785                 790                 795                 800
   CTG GTG GCT GGA GGC ATC GTA GCT GGG ATC TTC CTC ATT TTC ATC GAG     2448
   Leu Val Ala Gly Gly Ile Val Ala Gly Ile Phe Leu Ile Phe Ile Glu
                   805                 810                 815
   ATC GCC TAC AAG CGA CAC AAG GAT GCC CGT AGG AAG CAG ATG CAG CTG     2496
   Ile Ala Tyr Lys Arg His Lys Asp Ala Arg Arg Lys Gln Met Gln Leu
               820                 825                 830
   GCT TTT GCA GCC GTG AAC GTG TGG AGG AAG AAC CTG CAG GAT AGA AAG     2544
   Ala Phe Ala Ala Val Asn Val Trp Arg Lys Asn Leu Gln Asp Arg Lys
           835                 840                 845
   AGT GGT AGA GCA GAG CCC GAC CCT AAA AAG AAA GCC ACA TTT AGG GCT     2592
   Ser Gly Arg Ala Glu Pro Asp Pro Lys Lys Lys Ala Thr Phe Arg Ala
       850                 855                 860
   ATC ACC TCC ACC CTG GCC TCC AGC TTC AAG AGA CGT AGG TCC TCC AAA     2640
   Ile Thr Ser Thr Leu Ala Ser Ser Phe Lys Arg Arg Arg Ser Ser Lys
   865                 870                 875                 880
   GAC ACG AGC ACC GGG GGT GGA CGC GGC GCT TTG CAA AAC CAA AAA GAC     2688
   Asp Thr Ser Thr Gly Gly Gly Arg Gly Ala Leu Gln Asn Gln Lys Asp
                   885                 890                 895
   ACA GTG CTG CCG CGA CGC GCT ATT GAG AGG GAG GAG GGC CAG CTG CAG     2736
   Thr Val Leu Pro Arg Arg Ala Ile Glu Arg Glu Glu Gly Gln Leu Gln
               900                 905                 910
   CTG TGT TCC CGT CAT AGG GAG AGC                                 2760
   Leu Cys Ser Arg His Arg Glu Ser
           915                 920
(2) INFORMATION FOR SEQ ID NO:3:
   (i) SEQUENCE CHARACTERISTICS:
      (A) LENGTH: 2760
      (B) TYPE: nucleic acid
      (C) STRANDEDNESS: double
   (ii) MOLECULAR TYPE: cDNA
   (vi) ORIGINAL SOURCE: mouse
      (A) ORGANISM: brain
      (D) TOPOLOGY: linear
   (xi) SEQUENCE DESCRIPTION: SEQ ID NO:3:
   CGC GCT GCC TGC GAC CCC AAG ATT GTC AAC ATC GGC GCG GTG CTG AGC      48
   Arg Ala Ala Cys Asp Pro Lys Ile Val Asn Ile Gly Ala Val Leu Ser
     1               5                  10                  15
   ACG CGC AAG CAC GAG CAG ATG TTC CGC GAG GCA GTA AAC CAG GCC AAT      96
   Thr Arg Lys His Glu Gln Met Phe Arg Glu Ala Val Asn Gln Ala Asn
                20                  25                  30
   AAG CGA CAC GGC TCT TGG AAG ATA CAG CTC AAC GCC ACT TCT GTC ACC     144
   Lys Arg His Gly Ser Trp Lys Ile Gln Leu Asn Ala Thr Ser Val Thr
            35                  40                  45
   CAC AAG CCC AAC GCC ATA CAG ATG GCC CTG TCA GTG TGT GAG GAC CTC     192
   His Lys Pro Asn Ala Ile Gln Met Ala Leu Ser Val Cys Glu Asp Leu
        50                  55                  60
   ATC TCT AGC CAG GTC TAC GCT ATC CTA GTT AGT CAC CCG CCT ACT CCC     240
   Ile Ser Ser Gln Val Tyr Ala Ile Leu Val Ser His Pro Pro Thr Pro
    65                  70                  75                  80
   AAC GAC CAC TTC ACT CCC ACC CCT GTC TCC TAC ACA GCT GGC TTC TAC     288
   Asn Asp His Phe Thr Pro Thr Pro Val Ser Tyr Thr Ala Gly Phe Tyr
                    85                  90                  95
   AGA ATC CCC GTC CTG GGG CTG ACT ACC CGA ATG TCC ATC TAC TCT GAC     336
   Arg Ile Pro Val Leu Gly Leu Thr Thr Arg Met Ser Ile Tyr Ser Asp
               100                 105                 110
   AAG AGC ATC CAC CTG AGC TTC CTT CGC ACC GTA CCA CCC TAC TCC CAC     384
   Lys Ser Ile His Leu Ser Phe Leu Arg Thr Val Pro Pro Tyr Ser His
           115                 120                 125
   CAG TCC AGC GTC TGG TTT GAG ATG ATG CGC GTC TAC AAC TGG AAC CAT     432
   Gln Ser Ser Val Trp Phe Glu Met Met Arg Val Tyr Asn Trp Asn His
       130                 135                 140
   ATC ATC CTG CTG GTC AGC GAT GAC CAC GAG GGC CGG GCA GCG CAG AAG     480
   Ile Ile Leu Leu Val Ser Asp Asp His Glu Gly Arg Ala Ala Gln Lys
   145                 150                 155                 160
   CGC CTG GAG ACG TTG CTG GAG GAG CGT GAG TCC AAG GCA GAG AAG GTG     528
   Arg Leu Glu Thr Leu Leu Glu Glu Arg Glu Ser Lys Ala Glu Lys Val
                   165                 170                 175
   CTG CAG TTT GAC CCA GGA ACC AAG AAT GTG ACG GCT CTG CTG ATG GAA     576
   Leu Gln Phe Asp Pro Gly Thr Lys Asn Val Thr Ala Leu Leu Met Glu
               180                 185                 190
   GCC CGG GAC CTG GAA GCC CGG GTC ATC ATC CTT TCT GCA AGC GAG GAC     624
   Ala Arg Asp Leu Glu Ala Arg Val Ile Ile Leu Ser Ala Ser Glu Asp
           195                 200                 205
   GAC GCT GCC ACC GTA TAC CGC GCA GCC GCG ATG CTG AAC ATG ACT GGC     672
   Asp Ala Ala Thr Val Tyr Arg Ala Ala Ala Met Leu Asn Met Thr Gly
       210                 215                 220
   TCT GGG TAC GTG TGG CTC GTC GGG GAG CGC GAG ATC TCT GGG AAT GCC     720
   Ser Gly Tyr Val Trp Leu Val Gly Glu Arg Glu Ile Ser Gly Asn Ala
   225                 230                 235                 240
   CTG CGC TAC GCT CCT GAC GGC ATC ATC GGA CTT CAG CTA ATC AAC GGC     768
   Leu Arg Tyr Ala Pro Asp Gly Ile Ile Gly Leu Gln Leu Ile Asn Gly
                   245                 250                 255
   AAG AAC GAG TCG GCC CAC ATC AGT GAC GCT GTG GGC GTG GTG GCA CAG     816
   Lys Asn Glu Ser Ala His Ile Ser Asp Ala Val Gly Val Val Ala Gln
               260                 265                 270
   GCA GTC CAC GAG CTC CTA GAA AAG GAG AAC ATC ACT GAT CCA CCG CGG     864
   Ala Val His Glu Leu Leu Glu Lys Glu Asn Ile Thr Asp Pro Pro Arg
           275                 280                 285
   GGT TGC GTG GGC AAC ACC AAC ATC TGG AAG ACA GGA CCA CTG TTC AAG     912
   Gly Cys Val Gly Asn Thr Asn Ile Trp Lys Thr Gly Pro Leu Phe Lys
       290                 295                 300
   AGG GTG CTG ATG TCT TCC AAG TAT GCA GAT GGA GTG ACT GGC CGT GTG     960
   Arg Val Leu Met Ser Ser Lys Tyr Ala Asp Gly Val Thr Gly Arg Val
   305                 310                 315                 320
   GAA TTC AAT GAG GAT GGG GAC CGG AAG TTT GCC AAC TAT AGT ATC ATG    1008
   Glu Phe Asn Glu Asp Gly Asp Arg Lys Phe Ala Asn Tyr Ser Ile Met
                   325                 330                 335
   AAC CTG CAG AAC CGC AAG CTG GTG CAA GTG GGC ATC TAC AAT GGT ACC    1056
   Asn Leu Gln Asn Arg Lys Leu Val Gln Val Gly Ile Tyr Asn Gly Thr
               340                 345                 350
   CAT GTC ATC CCA AAT GAC AGG AAG ATC ATC TGG CCA GGA GGA GAG ACA    1104
   His Val Ile Pro Asn Asp Arg Lys Ile Ile Trp Pro Gly Gly Glu Thr
           355                 360                 365
   GAG AAG CCT CGA GGA TAC CAG ATG TCC ACC AGA CTA AAG ATA GTG ACA    1152
   Glu Lys Pro Arg Gly Tyr Gln Met Ser Thr Arg Leu Lys Ile Val Thr
       370                 375                 380
   ATC CAC CAA GAA CCC TTC GTG TAT GTC AAG CCC ACA ATG AGT GAT GGC    1200
   Ile His Gln Glu Pro Phe Val Tyr Val Lys Pro Thr Met Ser Asp Gly
   385                 390                 395                 400
   ACA TGC AAA GAG GAG TTC ACA GTC AAT GGT GAC CCT GTC AAG AAG GTG    1248
   Thr Cys Lys Glu Glu Phe Thr Val Asn Gly Asp Pro Val Lys Lys Val
                   405                 410                 415
   ATC TGT ACG GGG CCT AAT GAC ACA TCC CCA GGA AGC CCA CGT CAC ACA    1296
   Ile Cys Thr Gly Pro Asn Asp Thr Ser Pro Gly Ser Pro Arg His Thr
               420                 425                 430
   GTG CCC CAG TGC TGT TAT GGC TTC TGC GTT GAC CTG CTC ATC AAG CTG    1344
   Val Pro Gln Cys Cys Tyr Gly Phe Cys Val Asp Leu Leu Ile Lys Leu
           435                 440                 445
   GCA CGG ACC ATG AAT TTT ACC TAC GAG GTG CAC CTT GTG GCA GAT GGC    1392
   Ala Arg Thr Met Asn Phe Thr Tyr Glu Val His Leu Val Ala Asp Gly
       450                 455                 460
   AAG TTT GGC ACA CAG GAG CGG GTA AAC AAC AGC AAC AAA AAG GAG TGG    1440
   Lys Phe Gly Thr Gln Glu Arg Val Asn Asn Ser Asn Lys Lys Glu Trp
   465                 470                 475                 480
   AAC GGA ATG ATG GGA GAG CTG CTC AGT GGT CAA GCA GAC ATG ATC GTG    1488
   Asn Gly Met Met Gly Glu Leu Leu Ser Gly Gln Ala Asp Met Ile Val
                   485                 490                 495
   GCT CCA CTG ACC ATT AAC AAT GAG CGT GCG CAG TAC ATA GAG TTC TCC    1536
   Ala Pro Leu Thr Ile Asn Asn Glu Arg Ala Gln Tyr Ile Glu Phe Ser
               500                 505                 510
   AAG CCC TTC AAG TAC CAG GGC CTG ACC ATT CTG GTC AAG AAG GAG ATC    1584
   Lys Pro Phe Lys Tyr Gln Gly Leu Thr Ile Leu Val Lys Lys Glu Ile
           515                 520                 525
   CCT CGG AGC ACA CTG GAC TCA TTC ATG CAG CCC TTT CAG AGC ACA CTG    1632
   Pro Arg Ser Thr Leu Asp Ser Phe Met Gln Pro Phe Gln Ser Thr Leu
       530                 535                 540
   TGG CTG CTG GTG GGG CTG TCA GTT CAT GTG GTG GCC GTG ATG CTG TAC    1680
   Trp Leu Leu Val Gly Leu Ser Val His Val Val Ala Val Met Leu Tyr
   545                 550                 555                 560
   CTG CTG GAC CGC TTC AGT CCC TTT GGC CGA TTT AAG GTG AAC AGC ACC    1728
   Leu Leu Asp Arg Phe Ser Pro Phe Gly Arg Phe Lys Val Asn Ser Thr
                   565                 570                 575
   AGT GAC CAG TCA AAT GCA CTG ACC CTG TCC TCT GCC ATG TGG TTT TCC    1776
   Ser Asp Gln Ser Asn Ala Leu Thr Leu Ser Ser Ala Met Trp Phe Ser
               580                 585                 590
   TGG GGC GTC CTG CTC AAC TCT GGC ATT GGG GAA GGT GCC CCC CGG AGT    1824
   Trp Gly Val Leu Leu Asn Ser Gly Ile Gly Glu Gly Ala Pro Arg Ser
           595                 600                 605
   TTC TCT GCT CGT ATC CTA GGC ATG GTG TGG GCT GGT TTT GCC ATG ATC    1872
   Phe Ser Ala Arg Ile Leu Gly Met Val Trp Ala Gly Phe Ala Met Ile
       610                 615                 620
   ATC GTG GCT TCC TAC ACT GCC AAC CTG GCA GCC TTC CTG GTG CTG GAT    1920
   Ile Val Ala Ser Tyr Thr Ala Asn Leu Ala Ala Phe Leu Val Leu Asp
   625                 630                 635                 640
   AGG CCT GAG GAG CGC ATC ACA GGC ATC AAT GAC CCC AGG CTC AGA AAC 1968
   Arg Pro Glu Glu Arg Ile Thr Gly Ile Asn Asp Pro Arg Leu Arg Asn
   645 650 655
   CCC TCA GAC AAG TTC ATC TAT GCA ACT GTA AAA CAG AGC TCT GTG GAT 2016
   Pro Ser Asp Lys Phe Ile Tyr Ala Thr Val Lys Gln Ser Ser Val Asp
   660 665 670
   ATC TAC TTC CGG AGG CAG GTG GAG TTG AGC ACC ATG TAC CGG CAC ATG 2064
   Ile Tyr Phe Arg Arg Gln Val Glu Leu Ser Thr Met Tyr Arg His Met
   675 680 685
   GAG AAG CAC AAT TAT GAG AGT GCA GCT GAG GCC ATC CAG GCT GTG CGG 2112
   Glu Lys His Asn Tyr Glu Ser Ala Ala Glu Ala Ile Gln Ala Val Arg
   690 695 700
   GAC AAC AAG CTC CAT GCC TTC ATC TGG GAC TCA GCT GTG CTG GAG TTT 2160
   Asp Asn Lys Leu His Ala Phe Ile Trp Asp Ser Ala Val Leu Glu Phe
   705 710 715 720
   GAG GCT TCA CAG AAG TGC GAT CTG GTG ACC ACG GGT GAG CTG TTC TTC 2208
   Glu Ala Ser Gln Lys Cys Asp Leu Val Thr Thr Gly Glu Leu Phe Phe
   725 730 735
   CCC TCC GGC TTT GGC ATC GGC ATG CGC AAG GAC AGC CCC TGG AAG CAA 2256
   Arg Ser Gly Phe Gly Ile Gly Met Arg Lys Asp Ser Pro Trp Lys Gln
   740 745 750
   AAT GTG TCC CTG TCC ATA CTC AAG TCC CAT GAG AAT GGC TTC ATG GAA 2304
   Asn Val Ser Leu Ser Ile Leu Lys Ser His Glu Asn Gly Phe Met Glu
   755 760 765
   GAC CTG GAT AAG ACA TGG GTT CGG TAT CAA GAA TGT GAC TCC CGC AGC 2352
   Asp Leu Asp Lys Thr Trp Val Arg Tyr Gln Glu Cys Asp Ser Arg Ser
   770 775 780
   AAT GCC CCT GCC ACC CTC ACT TTT GAG AAC ATG GCA GGG GTC TTC ATG 2400
   Asn Ala Pro Ala Thr Leu Thr Phe Glu Asn Met Ala Gly Val Phe Met
   785 790 795 800
   CTG GTG GCT GGA GGC ATC GTA GCT GGG ATC TTC CTC ATT TTC ATC GAG 2448
   Leu Val Ala Gly Gly Ile Val Ala Gly Ile Phe Leu Ile Phe Ile Glu
   805 810 815
   ATC GCC TAC AAG CGA CAC AAG GAT GCC CGT AGG AAG CAG ATG CAG CTG 2496
   Ile Ala Tyr Lys Arg His Lys Asp Ala Arg Arg Lys Gln Met Gln Leu
   820 825 830
   GCT TTT GCA GCC GTG AAC GTG TGG AGG AAG AAC CTG CAG GAT AGA AAG 2544
   Ala Phe Ala Ala Val Asn Val Trp Arg Lys Asn Leu Gln Asp Arg Lys
   835 840 845
   AGT GGT AGA GCA GAG CCC GAC CCT AAA AAG AAA GCC ACA TTT AGG GCT 2592
   Ser Gly Arg Ala Glu Pro Asp Pro Lys Lys Lys Ala Thr Phe Arg Ala
   850 855 860
   ATC ACC TCC ACC CTG GCC TCC AGC TTC AAG AGA CGT AGG TCC TCC AAA 2640
   Ile Thr Ser Thr Leu Ala Ser Ser Phe Lys Arg Arg Arg Ser Ser Lys
   865 870 875 880
   GAC ACG AGC ACC GGG GGT GGA CGC GGC GCT TTG CAA AAC CAA AAA GAC     2688
   Asp Thr Ser Thr Gly Gly Gly Arg Gly Ala Leu Gln Asn Gln Lys Asp
                   885                 890                 895
   ACA GTG CTG CCG CGA CGC GCT ATT GAG AGG GAG GAG GGC CAG CTG CAG     2736
   Thr Val Leu Pro Arg Arg Ala Ile Glu Arg Glu Glu Gly Gln Leu Gln
               900                 905                 910
   CTG TGT TCC CGT CAT AGG GAG AGC                                     2760
   Leu Cys Ser Arg His Arg Glu Ser
           915                 920

## Description

### Field of the Invention

This invention relates to novel proteins modified by protein engineering and genes coding the same, more specifically to proteins modified NMDA (N-methyl-D-aspartic acid) type glutamate receptors, which play a central role in nervous information transmission and genes (cDNAs) coding them.

### Background of the Invention

It has been suggested that glutamate receptors are main receptors of stimulant neurotransmitters in a central nervous system of a higher animal, play a central role in nervous information transmission at a synapse, and also are deeply concerned with appearance of synapse plasticity which is basically required for memory and learning and neuronal cell death caused by a disease such as cerebral ischemia and epilepsy. Thus, it is considered that clarifications of molecular structures and functions of the glutamate receptors are required to understand a transmission mechanism of nervous information in a center, a cerebral structure of higher order and a disease of a brain.

As in the case of receptors of acetylcholine and GABA (g-aminobutyric acid), the glutamate receptors are roughly classified into ion channel type glutamate receptors and G protein coupled type receptors (metabolism-controlling type receptors). Receptors which can affect long-term reinforcement of synapse transmission at a CA1 region of hippocampus of which the most advanced study has been made about synapse plasticity are two kinds of ion channel type glutamate receptors. That is, an NMDA type receptor having Ca2+ permeability and opening depending on membrane potential and a quisqualate/kinate type receptor (or a non-NMDA type receptor). While the non-NMDA type receptor performs general synapse transmission, the NMDA type receptor performs Ca2+ permeation when a high frequent stimulus which induces long-term reinforcement of synapse transmission is given. The Ca2+ permeation is inhibited by Mg²⁺ depending on membrane potential.

Although the glutamate receptors have important physiological functions as described above, molecular biological structures thereof had not been clarified for a long term. In recent years, Hollmann et al. cloned cDNA of a non-NMDA type glutamate receptor by means of a cDNA-expressing system using Xenopus oocytes ('Nature', 342, pp. 643 to 648 (1989)), and Nakanishi et al. cloned cDNA of a rat-G protein coupled type glutamate receptor ('Nature', 349, pp. 760 to 765 (1991)) and cDNA of a rat-NMDA type receptor ('Nature', 354, pp. 31 to 37 (1991)). Other kinds of genes of glutamate receptors also have been cloned. NMDA type receptors are classified into _{E} family (Nature, 358, 36-41 (1992)) and family (FEBS Lett., 300, 39-45 (1992)) from the viewpoint of homology of amino acid sequences. However, the molecular biological mechanisms thereof have not yet been clarified sufficiently under the present situation.

### Disclosure of the Invention

The present inventors have paid special attention to NMDA type glutamate receptors and made researches and studied intensively, and glutamate receptors modified by protein engineering have been obtained to accomplish the present invention.

That is, the characteristic features of the present invention reside in a modified glutamate receptor which is represented by an amino acid sequence described in SEQ ID NO:1, 2 or 3 of the sequense listing and a gene coding the same.

In the following, the present invention is explained in detail.

The modified glutamate receptor of the present invention has an amino acid sequence as shown in SEQ ID NO:1, 2 or 3 of the sequense listing.

The representatives of the modified glutamate receptors of the present invention are, as shown in SEQ ID NO:1, 2 and 3,

"_{E}2-N589Q" (SEQ ID NO:1) in which asparagine which is the 589th from the N terminal end of a _{E}2 subunit comprising 1456 amino acids (Nature, 358, pp.36-41(1992)) is replaced with glutamine,

"?1-N598Q" (SEQ ID NO:2) in which asparagine which is the 598th from the N terminal end of a ?1 subunit comprising 920 amino acids (FEBS Lett., 300, pp. 39 to 45 (1992)) is replaced with glutamine, and

"_{?}1-ZAZ" (SEQ ID NO:3) in which the 1726th to 1743rd base sequence of the above ?1 subunit is changed to a base sequence of ACCAGTGACCAGTCAAAT.

In the present invention, there may be included modified glutamate receptors in which amino acids or nucleic acids are partially removed, replaced or added within the range which does not impair activity as a glutamate receptor.

The glutamate receptor of the present invention and DNA fragments of the gene coding the same can be obtained by, for example, the following method.

First, cerebral tissues of a mammal such as mouse are homogenized in an aqueous solution containing guanidium thiocyanate or the like, and total RNAs are separated as precipitates by cesium chloride equilibrium density gradient centrifugation or sucrose density gradient centrifugation in accordance with the method of Chirgwin et al. (Biochemistry, 18, pp. 5294 to 5299 (1979)).

After separation, total RNAs are purified by extraction with phenol, precipitation with ethanol or the like, and the resulting RNAs are further purified by oligo (dT) cellulose column chromatography to isolate poly (A)-containing mRNAs (poly A+ mRNAs) including mRNAs of the desired glutamate receptor, whereby an mRNA fraction can be obtained.

The mRNA fraction prepared as described above and, for example, primer DNA as described in "FEBS Lett.", 272, pp. 73 to 80 (1990) are hybridized, and by using a reverse transcriptase and T4 DNA polymerase, double-stranded cDNAs are synthesized and prepared according to a conventional method.

Then, EcoRl linkers are added to both termini of cDNA strands.

The above cDNA strands are inserted into EcoRl cleavage sites of a λ phage vector such as λgt10 to obtain a recombinant λ phage DNA mixture.

By using the recombinant λ phage DNA mixture obtained as described above and using an in vitro packaging kit such as commercially available Gigapack Gold (trade name, produced by Promega Co.) according to an operating manual, the so-called in vitro packaging was carried out to obtain λ phage particles having the recombinant λ phage DNAs. The λ phage particles obtained are amplified by transforming host cells such as Escherichia coli according to a conventional method.

The obtained clones are transferred onto a nylon (trade name) film or a nitrocellulose film such as Gene Screening Plus (trade name, produced by Du Pont), and protein is removed in the presence of an alkali. The λ phage DNAs including cDNAs are hybridized with a ³²P-labeled probe prepared from fragments of cDNA of a mouse glutamate receptor which has been already cloned (FEBS Lett., 272, pp. 73 to 80 (1990)) whereby clones having extremely high possibility of coding the desired glutamate receptor can be narrowed down to several clones.

The proteins and genes coding the same of this invention are prepared by modifying the respective natural substances of the ∈2 subunit and the ζ1 subunit by protein or gene engineering. A method of such a modification is not particularly limited, and may be carried out specifically by the 2-step polymerase chain reaction (PCR) method by using a suitable synthetic oligonucleotide and DNA fragments derived from a plasmid of pBKSA_{E}2 (Nature, 358, pp. 36 to 41 (1992)) or pBKSAζ1 (FEBS Lett., 300, pp. 39 to 45 (1992)).

For these clones, activity test may be conducted by using translation system of Xenopus oocytes to obtain cDNA clones coding the desired glutamate receptor.

The cDNA obtained as described above can be expressed in, for example, transient in vitro protein translation system, specifically translation system using Xenopus oocytes as described in "Nature", 329, pp. 836 to 838 (1987), or in a host such as a CHO cell transformed by a plasmid for expressing a protein prepared by ligating such a cDNA to downstream of a promoter of a expression plasmid for animal cells. Then, the expressed protein is collected to obtain the glutamate receptor of the present invention according to a conventional method,.

### Brief Description of the Drawings

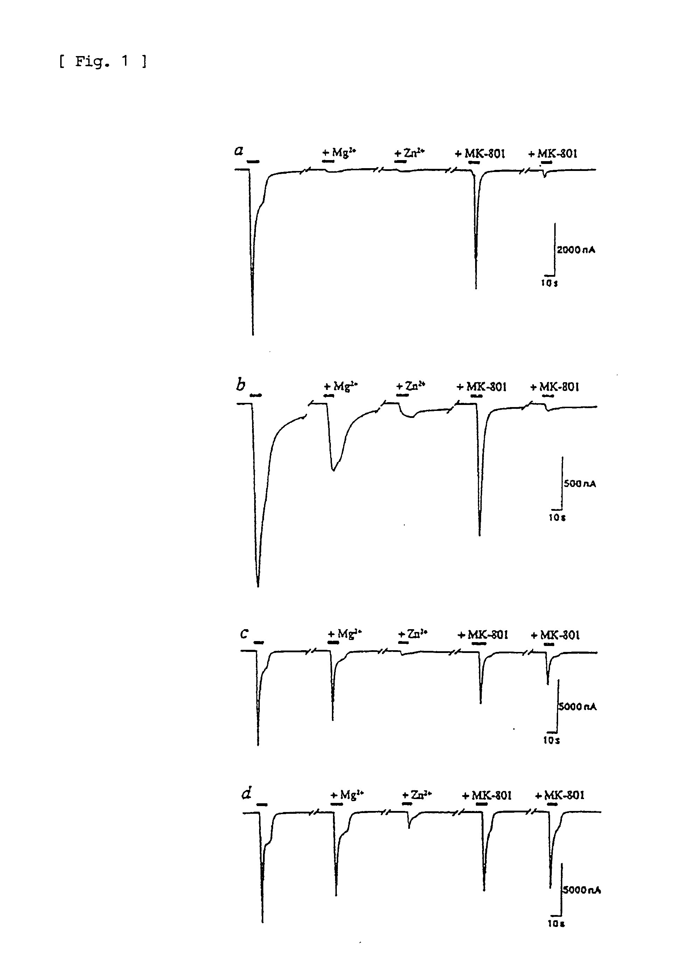

Fig. 1 shows a current response of the heteromeric NMDA receptor channel to 10µM L-glutamate + 10µM L-glycine at -70 mV of membrane potential in a frog standard Ringer's solution. In the figures, Figs. 1 (a), 1 (b), 1 (c) and 1 (d) represent an ∈2/ζ1 NMDA receptor channel, an ∈2/ζ1-N598Q NMDA receptor channel, an ∈2-N589Q/ζ1 NMDA receptor channel and an ∈2-N589Q/ζ1-N598Q NMDA receptor channel, respectively.

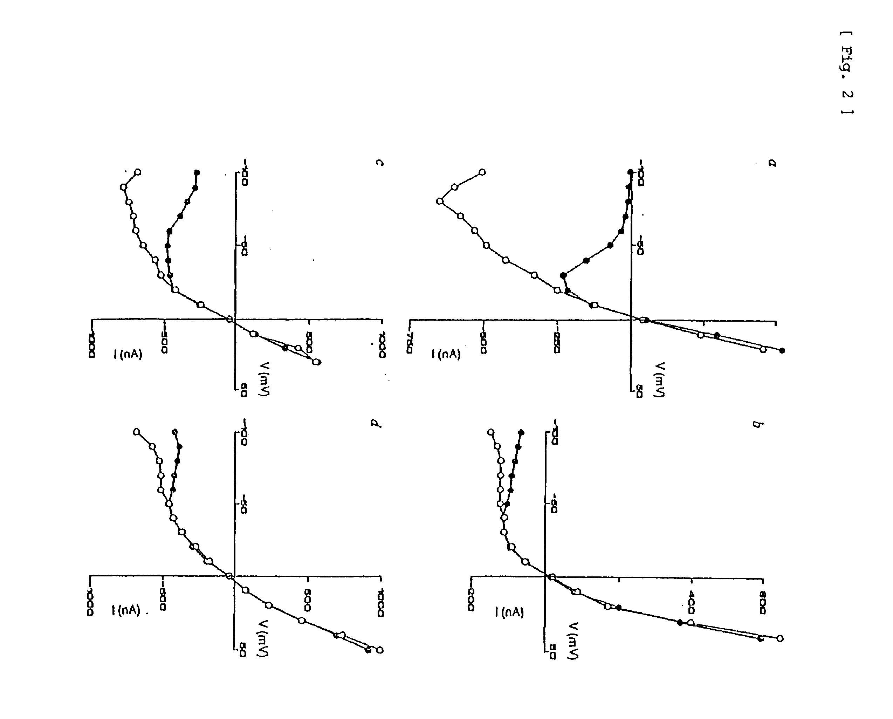

Fig. 2 shows current-voltage relationships of wild type and mutant heteromeric channels in the presence of 1 mM Mg²⁺ (•) and in the absence thereof (0). In the figures, Figs. 2(a), 2(b), 2(c) and 2(d) represent an ∈ 2/ζ1 NMDA receptor channel, an ∈2/ζ1-N598Q NMDA receptor channel, an ∈2-N589Q/ζ1 NMDA receptor channel and an ∈2-N589Q/ζ1-N598Q NMDA receptor channel, respectively.

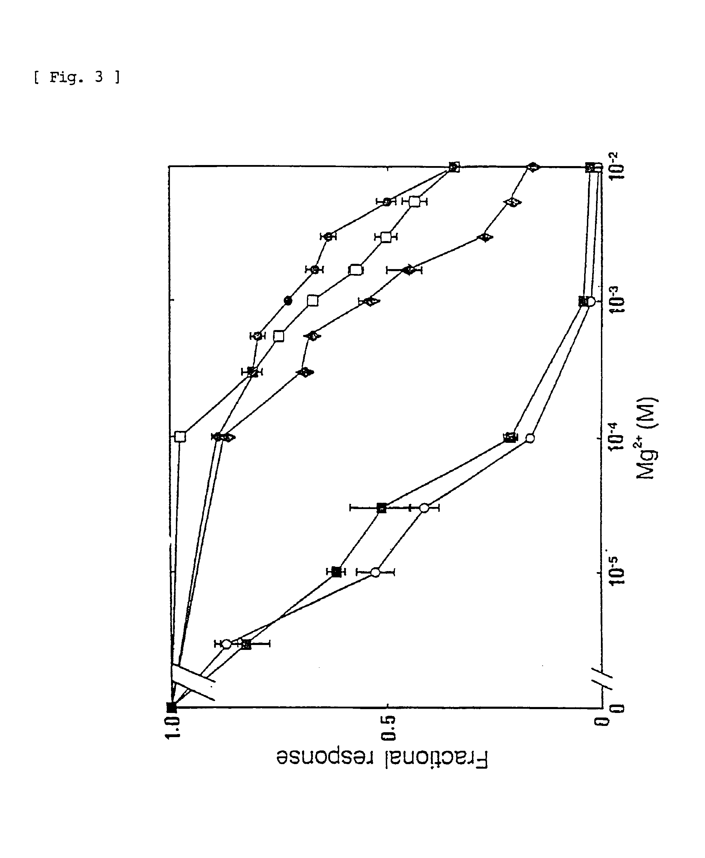

Fig. 3 shows the effects of Mg²⁺ concentrations on the response at -70 mV of membrane potential. In the figure, 0, •, ◆, o and represent an ∈2/ζ1 NMDA receptor channel, an ∈2/ζ1-N598Q NMDA receptor channel, an ∈2-N589Q/ζ1 NMDA receptor channel, an ∈2-N589Q/ζ1-N598Q NMDA receptor channel and an ∈2/ζ1-ZAZ (a mutant glutamate receptor shown in SEQ ID NO:3 of the sequense listing) NMDA receptor channel, respectively.

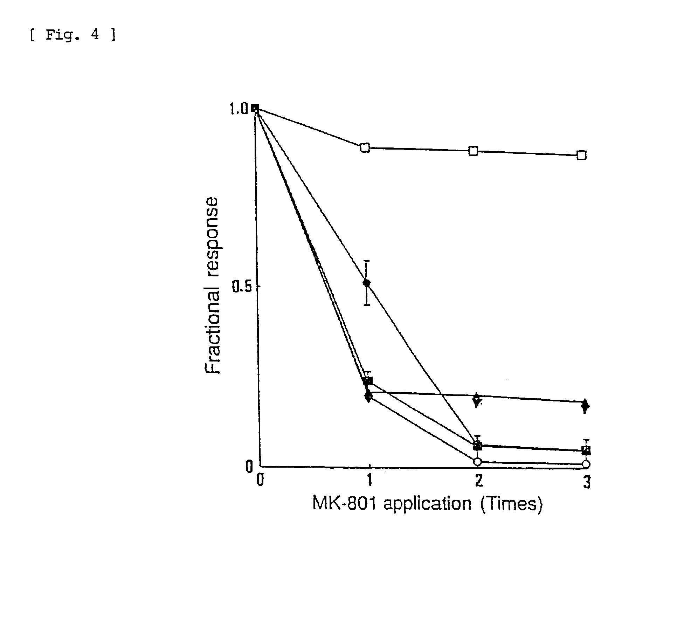

Fig. 4 shows inhibition after repetitive application of 1µM (+)-MK 801. In the figure, 0, •, ◆, □ and represent a ∈2/ζ1 NMDA receptor channel, an ∈ 2/21-N598Q NMDA receptor channel, an ∈2-N589Q/ζ1 NMDA receptor channel, an ∈2-N589Q/ζ1-N598Q NMDA receptor channel and an ∈2/ζ1-ZAZ NMDA receptor channel, respectively.

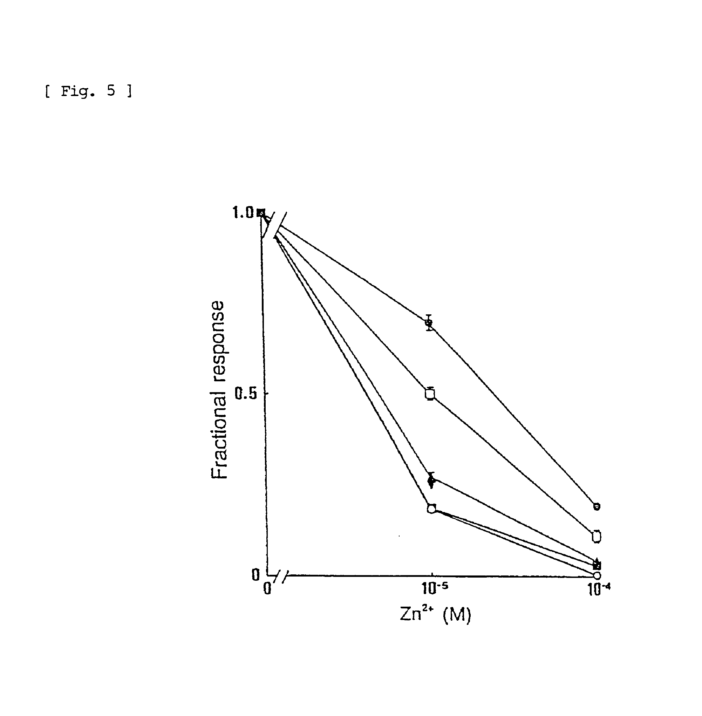

Fig. 5 shows the inhibition by various concentrations of Zn²⁺.

In the figures, 0, •, ◆, □ and represent an ∈2/ζ1 NMDA receptor channel, an ∈2/ζ1-N598Q NMDA receptor channel, an ∈2-N589Q/ζ1 NMDA receptor channel, an ∈2-N589Q/ζ1-N598Q NMDA receptor channel and an ∈2/ζ 1-ZAZ NMDA receptor channel, respectively.

### Best Mode for Carrying Out the Invention

The present invention is described in detail by referring to Examples, but the present invention is not limited by these Examples unless it is outside the scope thereof.

### Example 1

First, a cerebellum of ICR mouse was homogenized in an aqueous solution of guanidium thiocyanate, and then RNA was extracted, separated and purified according to the method of Chirgwin et al. (Biochemistry, 18, pp. 5294 to 5299 (1979)), and subjected to oligo (dT)-cellulose column chromatography according to the method of Aviv et al. (Proc. Natl. Acad. Sci. USA, 69, pp. 1408 to 1412 (1972)) to isolate poly (A)-containing mRNAs (poly A+ mRNAs) containing mRNAs of an NMDA type glutamate receptor.

By using a cDNA synthesizing kit produced by Bethesda Research Laboratories Co., cDNAs having double strand were prepared from the poly A+ mRNAs. Both termini of the cDNAs were blunt-ended with T4 DNA polymerase, and the cDNAs were methylated by using EcoRI-methylase. Then, EcoRl linkers were added to both termini of the cDNAs by ligase to prepare cDNA fragments having EcoRl restriction site at both termini by digestion with EcoRl.

The cDNAs obtained as described above were subjected to 1.5 % agarose gel electrophoresis to select and collect cDNAs having a size of 0.5 Kb or more.

The obtained cDNA fragments having a size of 0.5 Kb or more were ligated to λgt10 phage DNAs with ligase, and the cDNAs were inserted into an EcoRl cleaved site of λgt10 to constitute a λgt10 cDNA library according to a conventional method. By using the λgt10 cDNA library obtained and using an in vitro packaging kit Gigapack Gold (trade name, produced by Promega Co.) according to an operating manual, in vitro packaging was carried out to obtain λ phage particles having recombinant λ phage DNAs. The λ phage particles obtained were amplified by transforming Escherichia coli of host cells according to a conventional method.

The obtained clones was transferred onto a nylon (trade name) film Gene Screening Plus (trade name, produced by Du Pont), and protein was removed in the presence of an alkali. Then screening of the cDNA library was carried out. The Kpnl/Hindlll DNA fragments (1383 bp; previously cloned cDNA fragments of mouse glutamate receptors) of a pGRA19 plasmid (Nature, 357, 70-74 (1992)) and cDNAs of mouse a1 and a2 subunits (FEBS Lett., 272, 73-80 (1990)) were labeled with ^{32p} to prepare probes and plaque hybridization was performed in the presence of 30 % of formaldehyde at 37 _{°} C.

Site-directed mutagenesis was carried out by using a suitable synthesized oligonucleotide and DNA fragments derived from pBKSA ∈2 plasmid (Nature, 358, pp. 36-41(1992)) and pBKSA ζ1 plasmid (FEBS, Lett., 300, pp. 39 to 45 (1992)) according to the two-step polymerase chain reaction (PCR) method. The resulting modified 353 base pairs Cfr101/Sphl and 322 base pairs Fspl/Blnl DNA fragments obtained by amplifying according to the PCR were substituted for the corresponding segments of pBKSA ∈2 and pBKSA ζ1, respectively. The nucleotide sequences of the constructed plasmids are different from those of the original plasmids as follows.

pBKSA_{E}2-N589Q: in the ∈2 subunit described in "Nature", 358, pp.36-41(1992), asparagine which is 589th amino acid residue from the N-terminal is modified to glutamine, 1765th nucleotide A is modified to C and 1767th nucleotide is modefied to G, respectively. The amino acid sequence and base sequence are shown in SEQ ID NO:1 of the sequense listing.

pBKSAζ1-N598Q : in the ζ1 subunit described in "FEBS Lett.", 300, pp. 39 to 45 (1992), asparagine which is an amino acid at the 598th from the N-terminal is modified to glutamine, 1792nd nucleotied A is modified to C and 1794th nucleotide C is modified to G, respectively. The amino acid sequence and base sequence are shown in SEQ ID NO:2 of the sequense listing.

pBKSAζ1-ZAZ: in the ζ1 subunit described in "FEBS Lett.", 300, pp. 39 to 45 (1992), the base sequence of 1726th - 1743rd is modified to ACCAGTGACCAGTCAAAT. The amino acid sequence and base sequence are shown in SEQ ID NO:3 of the sequense listing.

Next, by using the pBKSA _{E}2 and pBKSA ?1 plasmids and derivatives thereof cut by suitable restriction enzymes as template and using T3 RNA polymerase produced by BRL Co., _{E}2, mRNA which is specific to ?1 and their derivatives were synthesized in vitro, respectively.

Transcription was carried out in a solution containing ATP, TTP and CTP at a concentration of 0.5 mM, respectively and 0.1 mM GTP in the presence of 0.5 mM dinucleotide 7mGpppG having a cap structure (in which two Gs were linked at 5' and 7-site and one of each guanine was methylated, produced by P-L Biochemicals Co.).

The wild type or modified _{E}2 subunit-specific mRNAs (not more than 19 ng/oocyte) and the wild type or modified ?1 subunit-specific mRNAs (not more than 13 ng/oocyte) were injected into the Xenopus oocytes.

The oocytes used were obtained from mature female Xenopus and separated into one oocyte in a Barth medium by using sharp tweezers and scissors. Ten µl of the mRNA solution was injected into about 100 oocytes in an amount of 50 to 100 nl per one oocyte. The mRNAs were injected under a microscopic observation by using a capillary and a micromanipulator. After the injection, the oocytes were incubated at 19 °C for one day in a Barth medium ("The Control of Gene Expression in Animal Development", Clarendon, Oxford (1974)) containing 0.1 mg/ml of gentamicin. Then, the oocytes were allowed to stand in 1 mg/ml of collagenase at room temperature for 1 hour, and then extracellular skins were removed under a microscope by using sharp tweezers. These oocytes were resuspended in the Barth medium containing 0.1 mg/ml of gentamicin, incubated again at 19 °C for one day and then used for an electrophysiological test.

The electrophysiological test was carried out by a conventional micropipet voltage clamp method. Oocytes generally have a membrane potential of about -30 to -40 mV. First, while a frog standard Ringer's solution (115 mM NaCI, 2.5 mM KCI, 1.8 mM CaC1₂ and 10 mM HEPES-NaOH (pH: 7.2)) was flown in a chamber, two glass microelectrodes charged with 3M KCI were stuck in the oocytes, and the membrane potential was fixed to -70 mV. In these oocytes, an agonist (NMDA) is linked to a glutamate receptor expressed on a cell membrane and an ion channel is opened, the oocytes have an action of returning membrane potential to an original value, whereby inflow and outflow of ions are caused. In this experiment system, flow of these ions were detected, and opening and closing of the ion channel were observed.

Fig. 1 shows a current response of the heteromeric NMDA receptor channel to 10µM L-glutamate and 10µM L-glycine at -70 mV membrane potential in a frog standard Ringer's solution. The wild type ∈2/3ζ1 NMDA receptor channel was strongly inhibited by 1 mM Mg2 +²⁺, 100µM Zn²⁺ and 1µM (+)-MK-80₁(an open channnel blocker of the NMDA type receptor channnel). To the contrary, the modified ∈2/ζ1-N598Q channel showed a great current response even in the presence of 1 mM Mg^{2+.} However, the response was suppressed effectively by 100M Zn²⁺ and 1µM (+)-MK-80₁. Similarly, in the modified _{E}2-N589Q, sensitivity to Mg²⁺ inhibition was decreased without changing sensitivty to Zn^{2+.} In comparison with the ∈2/ζ1 and ∈2/ζ1-N598Q channels, the ∈2-N589Q/ζ1 channel showed a great current response after repetitive application of (+)-MK-801. The heteromeric ∈2-N589Q/ζ1 -N598Q channel showed strong resistance to Mg²⁺ and (+)-MK-801 inhibitions, but still had sensitivity to Zn²⁺.

In order to minimize effects of a secondary activated Ca2+ dependent CI- current, influences of these modified proteins on Mg²⁺ inhibition were measured more quantitatively in a Ba2+ Ringer's solution. Figs. 2a to 2d each represent current-voltage curves of the wild type and modified heteromeric channels in the presence of 1 mM Mg²⁺ (•) and in the absence thereof (0). As observed in the case of the NMDA type receptor channel, Mg²⁺ inhibited a current response to the wild type ∈2/ζ 1 channel depending on voltage (Fig. 2a). Sensitivity of the heteromeric channel to Mg²⁺ was decreased greatly by mutation at an asparagine residue(s) of one or both of the subunits, and the modified channel retained activity even at -100 mV membrane potential (Figs. 2b to 2d). Inhibition degrees at various Mg²⁺ concentrations were compared at -70 mV membrane potential (Fig. 3). Activities of the wild type ∈2/ζ1 channel and the ∈2/ζ 1-ZAZ channel were decreased by 50 % by not more than 20µM Mg²⁺ and suppressed almost completely by not more than 1 mM Mg²⁺ which is a physiological concentration. On the other hand, about 100-fold concentration of Mg²⁺ was required to depress the modified ∈2/ζ1-N598Q, ∈2-N589Q/ζ1 and ∈2-589Q/ζ1-N598Q channels.

By administering 1µM (+)-MK-801 which is an open channel blocker of the NMDA type receptor channel repeatedly, the wild type ∈2/ζ1 channel was suppressed almost completely (Fig. 4). Similarly, activities of the modified ∈2/ζ1-N598Q and ∈2-N589Q/ζ1 channels were strongly inhibited by continuous application of
(+)-MK-801. On the other hand, the modified ∈2-N589Q/ζ1-N598Q channel retained high activities even after
(+)-MK-80₁ was applied three times repeatedly.

Effect of Zn2 +²⁺ which had been reported to induce non-competitive inhibition to the NMDA type receptor not depending on voltage was examined. Sensitivity of the heteromeric channel to 10µM Zn²⁺ was decreased a little by modification. However, the modified channel was strongly inhibited by 100µM Zn^{2+ -} (Fig. 5).

These results suggested that the NMDA type receptor channels having different functions were formed by modification of the subunits.

### Industrial Applicability

The genes for NMDA type glutamate receptor of the present invention are extected to be not only useful for clarifying nervous information transmission at a synapse, expression of synapse plasticity which is basically required for memory and learning and neuronal cell death caused by a disease such as cerebral ischemia and epilepsy and understanding a transmission mechanism of nervous information in a center, a cerebral structure of higher order and a disease of a brain, but also useful for therapy of genetic diseases and preparation of novel pharmaceuticals (e.g. screening of an agonist or an antagonist).

## Claims

1. A protein having an amino acid sequence selected from the group consisting of amino acid sequences represented in SEQ ID NO:1, 2 and 3.

2. A gene coding for a protein described in Claim 1.

3. A gene according to Claim 3, having a base sequence selected from the group consisting of base sequences represented in SEQ ID NO:1, 2 and 3.
